# FASCICULE DE BREVET EUROPEEN

(11) **EP 3 609 550 B1**
(45) Date de publication et mention de la délivrance du brevet: **09.06.2021**
(21) Numéro de dépôt: 18727042.6
(22) Date de dépôt: 06.04.2018
(51) Int. Cl.: A61M 1/00

(54) **DISPOSITIF DE TRAITEMENT DES CELLULES GRAISSEUSES PRÉLEVÉES SUR UN PATIENT ET DESTINÉES À UNE GREFFE**
VORRICHTUNG ZUR BEHANDLUNG VON AUS EINEM PATIENTEN ENTFERNTEN UND ZUR TRANSPLANTATION BESTIMMEN FETTZELLEN
DEVICE FOR TREATING FAT CELLS TAKEN FROM A PATIENT AND INTENDED FOR A TRANSPLANT

(30) Priorité: 11.04.2017 FR 1770365
(43) Date de publication de la demande: 19.02.2020
(73) Titulaire: Establishment Labs S.A., 20113 Alajuela (CR)
(72) Inventeur: TAIZOU, Najib, 47250 Sainte Geme-Martaillac (FR)
(74) Mandataire: Haseltine Lake Kempner LLP
(86) Numéro de dépôt international: PCT/FR2018/000076
(87) Numéro de publication internationale: WO 2018/189431

(56) Documents cités:
- WO-A1-2014/039697
- US-A1- 2012 271 254
- US-A1- 2014 081 237

## Description

### Domaine de l'invention

La présente invention concerne un dispositif et un procédé de traitement des cellules graisseuses prélevées sur un patient et destinées à une greffe. Plus précisément, il s'agit d'un dispositif et d'un procédé par aspiration permettant de séparer les cellules graisseuses viables des résidus et éléments qui ne sont pas greffables.

### Brève description de l'antériorité

Le lipofilling ou autogreffe de tissus graisseux est une technique qui consiste à prélever des petites quantités de graisse sur un patient pour les réinjecter sur le même patient afin de combler ou de remodeler une partie du corps.

Après le prélèvement initial de la graisse, il convient de la nettoyer afin de séparer les cellules graisseuses intactes des substances autres qui ne sont pas greffables (sang, huile provenant de l'explosion de cellules de mauvaise qualité,...).

Pour procéder à cette opération de nettoyage, différents dispositifs ont été développés. Le produit commercialisé sous la marque Puregraft consiste en une poche souple comprenant une membrane filtrante centrale séparant deux zones. La poche présente une entrée de fluide provenant du corps d'un patient et une sortie de fluide pour son utilisation dans une autre intervention chirurgicale. La graisse prélevée est filtrée à travers la membrane de filtration, la graisse de mauvaise qualité et les résidus sont isolés et évacués. L'utilisation de ce dispositif est cependant relativement long et finalement peu rentable.

Le produit commercialisé sous la marque Revolve par la société Lifecell permet de prélever, traiter et extraire la graisse dans un unique réservoir. Un système permet le chargement des seringues pour la réinjection directement à partir du réservoir. Le dispositif fonctionne grâce à une poignée qui doit être activée par rotation après avoir ajouté une solution de rinçage. Mais cette action nécessite une intervention humaine et monopolise donc du personnel.

Le brevet US2008/0281256 décrit un dispositif de centrifugation pour réaliser une liposuccion suivie d'une lipoinjection. La centrifugeuse comprend une pompe à air pour l'aspiration et la compression. Outre que ce dispositif nécessite de nombreuses manipulations, il est avéré que lors d'une centrifugation, les cellules extraites sont soumises à des contraintes physiques très importantes, réduisant leur taux de survie Un autre dispositif pour réaliser une liposuccion est décrite dans WO 2014/039697.

La présente invention vise à pallier tous ces inconvénients en proposant un dispositif peu encombrant permettant un traitement des graisses rapide, simple et optimal, tout en préservant l'intégrité des cellules graisseuses. Le dispositif proposé est par ailleurs peu couteux et très rentable.

### Sommaire de l'invention

La présente invention permet d'améliorer la technique en proposant un dispositif mécanique pour le traitement de tissus adipeux chez un patient au cours d'une procédure de transfert de tissus adipeux.

Il s'agit d'un dispositif de traitement de tissus adipeux connecté à un moyen d'aspiration, constitué d'une chambre de traitement étanche comprenant un moyen de connexion audit moyen d'aspiration, un moyen d'entrée desdits tissus adipeux avant traitement, un moyen de filtration et au moins un moyen de sortie desdits tissus adipeux après traitement, ledit moyen de filtration comprenant une paroi extérieure de forme conique comportant une base resserrée fixée sur une hélice et un moyen de tamisage intérieur relié au moyen de sortie, ladite hélice étant soumise à la dépression d'air créée par le moyen d'aspiration et constituant un moyen d'actionnement mécanique rotatif dudit moyen de filtration.

Préférentiellement le moyen d'entrée desdits tissus adipeux constitue un moyen d'entrée d'un fluide de nettoyage.

Avantageusement la chambre de traitement comporte un moyen de sortie du fluide de nettoyage.

Selon un mode de réalisation, le moyen de tamisage est constitué d'un maillage, chacune des mailles constituant le maillage ayant une dimension comprise entre 500 microns et 1 mm.

Selon une variante, le moyen de filtration comporte au moins un moyen de malaxage.

De manière avantageuse, la chambre de traitement comprend un train épicycloïdal constituant un moyen d'agitation desdits tissus adipeux.

Selon un autre mode réalisation, la chambre de traitement est reliée par un moyen de connexion à un moyen de contrôle électronique du processus de traitement des tissus adipeux.

De manière préférentielle, la chambre de traitement comporte un moyen d'attache.

### Brève description des dessins

Dans cette description, on se réfère aux dessins annexés sur lesquels :

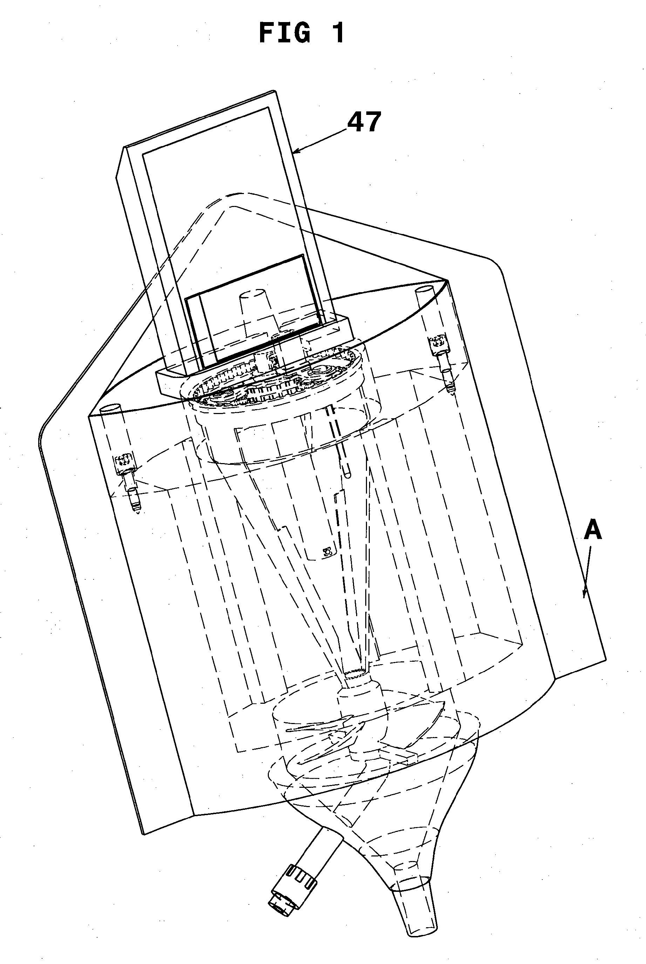

- Figure 1 : Représente une vue de profil du dispositif.

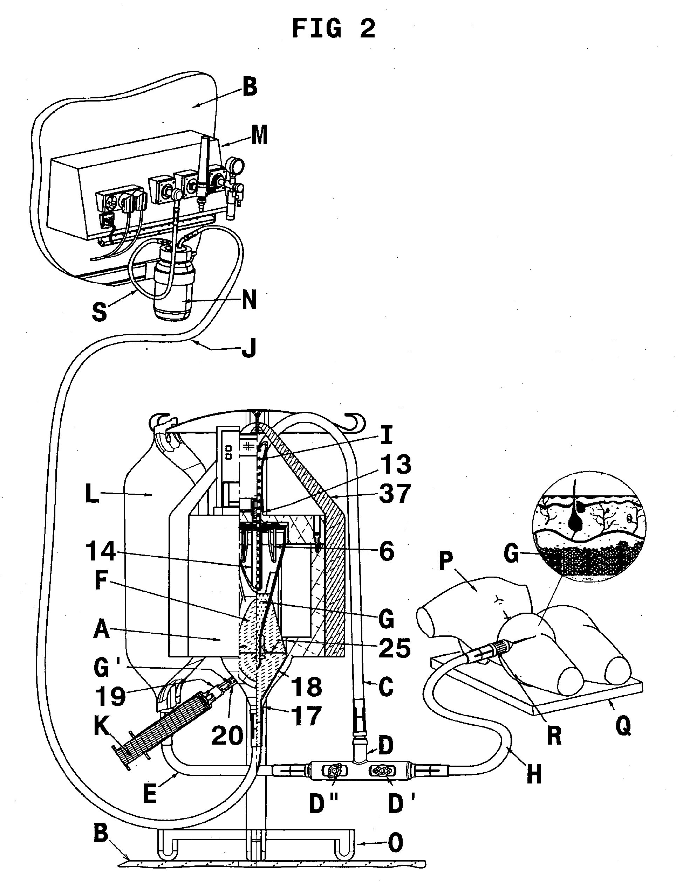

- Figure 2 : Représente une vue de la mise en place et la préparation du dispositif avant l'opération.

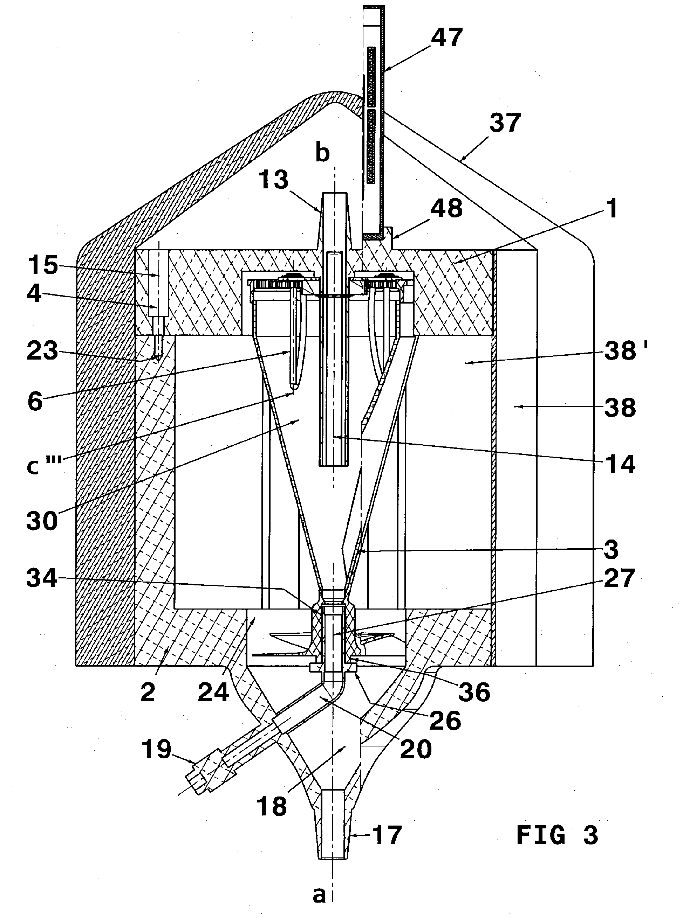

- Figure 3 : Représente une vue en coupe du dispositif.

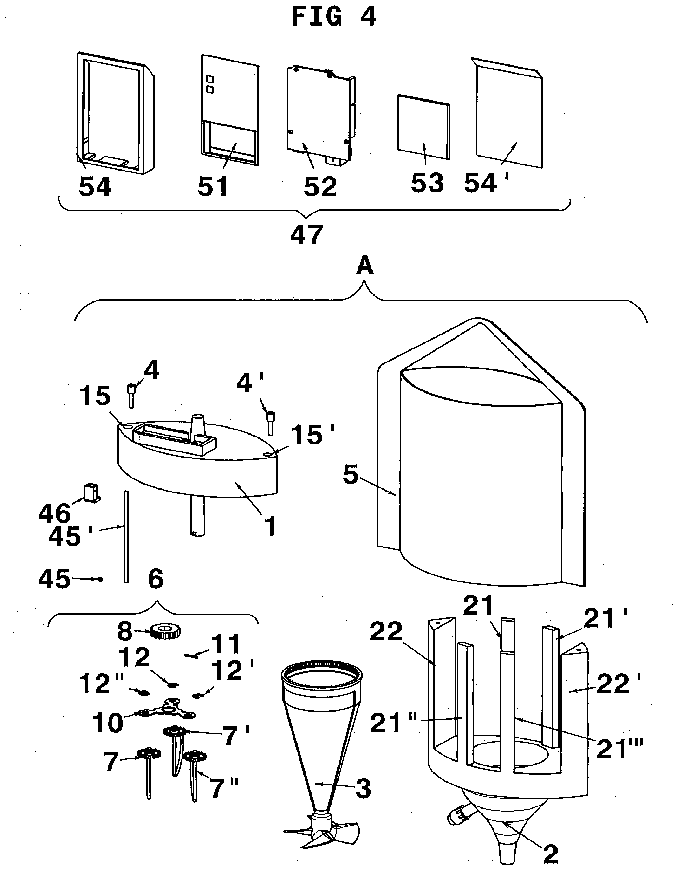

- Figure 4 : Représente une vue éclatée de profil du dispositif.

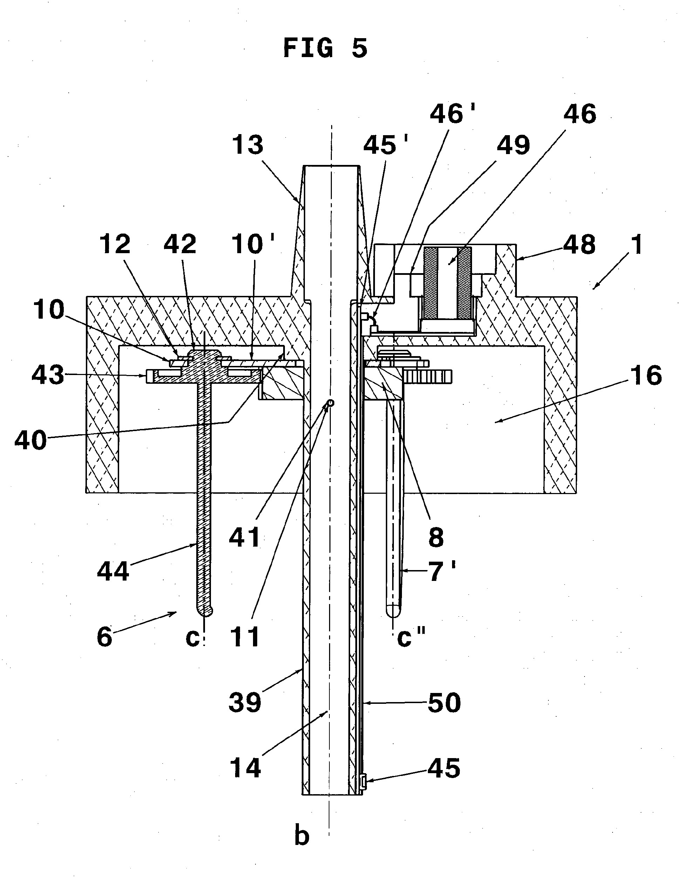

- Figure 5 : Représente une vue en coupe du bloc d'admission avec un train d'engrenage épicycloïdal.

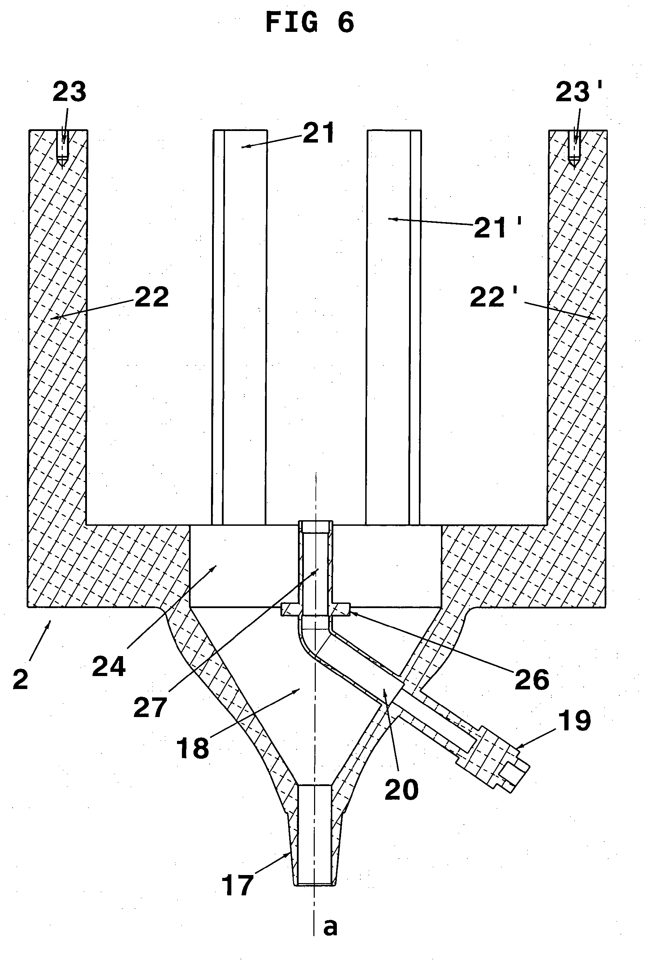

- Figure 6 : Représente une vue en coupe du bloc de refoulement.

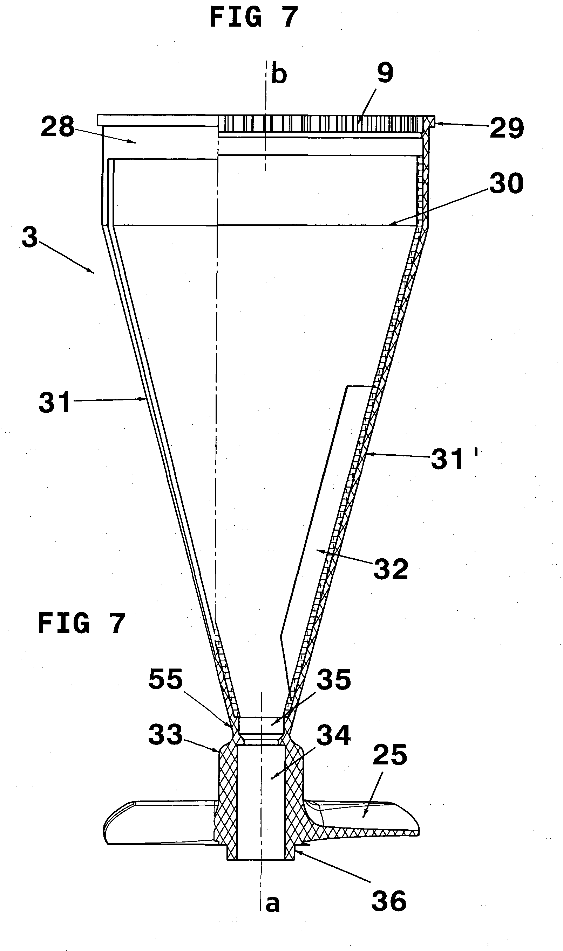

- Figure 7 : Représente une vue en coupe du filtre.

### Description détaillée des modes préférés de réalisation

Selon un mode préféré de réalisation, le dispositif est une chambre de traitement (A) mécanique et étanche, qui transforme en énergie mécanique une énergie de nature différente et assure ainsi la purification par centrifugation du volume de tissus adipeux. Le fonctionnement de la chambre de traitement (A) repose sur la transformation d'un débit ou flux d'air en force centrifuge.

La chambre de traitement (A) désigne l'ensemble des pièces servant ou pouvant servir à piloter le processus de traitement par centrifugation du tissu adipeux. Cette chambre de traitement (A) présente une zone centrale, une zone supérieure et une zone inférieure, ces trois zones sont horizontales. Pour faciliter la compréhension du dispositif, la notion de bloc technique est à définir. Un bloc technique est une masse solide formant un corps unitaire ou un ensemble de corps solidaires entre eux. Lesdits blocs sont composés d'organes tel que des gorges, des épaulements, des assises, des logements, perçages ou autres, qui assurent simultanément plusieurs fonctions comme par exemple "permettre une rotation" ou "permettre le passage d'un liquide", etc.

Ladite chambre de traitement (A) est composée de deux blocs techniques immobiles nommés bloc d'admission (1) et bloc de refoulement (2), liés par deux vis (4,4'). Cette chambre de traitement (A) est aussi composée d'un bloc technique mobile nommé filtre (3).

Ladite chambre de traitement (A) assure l'essentiel de l'étanchéité grâce une enveloppe (5) collée ou soudée thermiquement avec les blocs d'admission et de refoulement (1,2).

Ledit bloc d'admission (1) situé dans la zone supérieure de la chambre de traitement (A), a pour fonction principale de fournir suffisamment la chambre de traitement (A) en air et en tissus adipeux.

Ledit bloc d'admission (1) comprend une partie à l'extérieur de la chambre de traitement (A), et une partie à l'intérieur de la chambre de traitement (A), chaque parties présentes une zone centrale, et une zone de chaque coté, ces trois dernières zones sont sensiblement verticales. Ledit bloc d'admission (1) comprend sur sa zone extérieure a la chambre de traitement (A) un moyen d'entrée nommé orifice d'entrée conique (13) positionné au centre, qui fait communiquer un conduit fluidique (14) avec l'extérieur. Le bloc d'admission (1) comprend aussi sur sa partie extérieure, deux perçages à fonds plats (15,15') pour le passage et le serrage des deux vis à tête cylindrique (4,4').

Le bloc d'admission (1) comprend sur sa zone a l'intérieur de la chambre de traitement (A), un logement (16) dans lequel se loge le filtre (3). Ledit bloc d'admission (1) comprend aussi sur sa zone située à l'intérieur du dispositif un conduit fluidique vertical (14), de forme cylindrique. Ledit conduit fluidique (14) vertical achemine le tissu prélevé (I) à traiter et un fluide de nettoyage (L), de l'orifice d'entrée conique (13) jusqu'au centre du filtre (3). Le bloc d'admission (1) peut comprendre une ouverture avec un filtre antibactérien pour laisser passer plus d'air dans la chambre de traitement (A).

Le bloc de refoulement (2) situé dans la zone inférieure de ladite chambre de traitement (A), a pour fonction principale de refouler ou évacuer le filtrat (F) et les tissus adipeux (G) présents dans la chambre de traitement (A).

Ledit bloc de refoulement (2) comprend une partie à l'extérieur de la chambre de traitement (A) et une partie a l'intérieur de la chambre de traitement (A), chaque partie présente une zone centrale, et une zone de chaque coté, ces trois dernières zones sont sensiblement verticales.

Ledit bloc de refoulement (2) comprend sur sa partie extérieure à la chambre de traitement un moyen de connexion nommé orifice de sortie conique (17) positionné au centre et qui fait communiquer un conduit fluidique (18) avec l'extérieur. Ledit bloc de refoulement (2) comprend aussi sur sa partie extérieure, un orifice de sortie luer-lock (19) pour établir une connexion sans fuite entre un moyen de sortie nommé conduit fluidique (20) et un raccord mâle de seringue (K).

Ledit bloc de refoulement (2) comprend sur sa partie intérieure ledit conduit fluidique (18) vertical, qui évacue l'air et le filtrat (F) présents dans la chambre de traitement (A) vers l'extérieur. Le bloc de refoulement (2) comprend aussi sur sa partie intérieure ledit conduit fluidique (20) qui dirige le tissu adipeux (G) se trouvant dans le filtre (3) jusqu'à l'orifice luer-lock (19). Ledit bloc de refoulement (2) comprend encore quatre colonnes simples (21,21',21",21"') au centre, qui supportent le bloc d'admission (1) et empêchent l'enveloppe (5) de se déformer sous l'effet de la dépression et de gêner le bon fonctionnement du dispositif, puis deux colonnes (22,22') sur les extrémités, liées au bloc d'admission (1) grâce à des trous taraudés (23,23') aux dimensions des deux vis (4,4'). Ledit bloc de refoulement (2) aussi comprend sur sa partie intérieure un logement (24) dimensionné pour permettre la rotation de l'hélice (25) du filtre (3). Ledit logement (24) est doté d'une assise (26) qui supporte le filtre (3). Ledit logement (24) est de forme cylindrique, il sert notamment d'orifice d'entrée du filtrat (F) à un conduit fluidique (18). Le conduit fluidique (18) part du logement (24) jusqu'à l'orifice de sortie conique (17). Les dimensions du conduit (18) déterminent le débit général, donc, la vitesse de rotation du filtre (3). Ledit conduit fluidique (20), de forme cylindrique, est au centre du conduit (18), il part d'un orifice d'entrée (27) au centre du logement (24), jusqu'a l'orifice de sortie luer-lock (19). L'orifice d'entrée (27) guide la rotation du filtre (3).

Ledit filtre (3) situé dans la zone centrale de la chambre de traitement (A), est un moyen de filtration supporté par le bloc de refoulement (2), est guidé en rotation par les deux blocs (1,2). Ledit filtre (3) a pour fonction principale la centrifugation ou la séparation des composés d'un mélange en fonction des différences de densité des particules solides en suspension dans un fluide, en les soumettant à une force centrifuge. Ledit filtre (3) présente une zone centrale, une zone supérieure et une zone inférieure, ces trois zones sont horizontales.

Ledit filtre (3) comporte, sur sa zone supérieure, un anneau (28) et un épaulement (29), prévu pour accueillir des éléments de mesures de vitesses de rotation.

Ledit filtre (3) comporte sur sa zone centrale un tamis (30), qui piège le tissu (G) et évacue le filtrat (F). Le volume confiné des tissus adipeux (G) à l'intérieur du tamis (30), est autrement nommé retentât (G). La forme conique du moyen de tamisage intérieur (30) permet de diriger le tissu adipeux (G) en son sein, et de bénéficier au moins de 80 % à 95% du volume traité disponible. Le tamis (30) peut être dimensionné avec un maillage allant dans une plage de 500 microns à 1mm. Le tamis (30) peut être de toute conception, toute structure poreuse. Le tamis (30) peut être collé, soudé, ou moulé au filtre (3) d'une base resserrée (55) à un anneau (28). Le retentât (G) disponible dans le tamis (30) peut être dans une gamme ayant une limite inférieure de 50 centimètres cubes, et une limite supérieure de 350 centimètres cubes. Ledit tamis (30) est rigidifié par deux renforts conique (31,31') qui supportent aussi l'anneau (28) et forment la paroi du filtre (3). Ledit filtre (3) comporte aussi deux moyens de malaxage nommé malaxeurs (32), qui ont pour fonction d'agiter les graisses (G) lors de la rotation du filtre (3). Les deux malaxeurs (32) sont placés sur les deux renforts (31,31'). Les deux malaxeurs (32) permettent d'améliorer le nettoyage et le transfert des matières organiques vers le tamis (30).

Le filtre (3) comporte sur sa zone inférieure, un moyen d'actionnement mécanique rotatif dudit moyen de filtration, une hélice (25) à quatre pales qui transforme l'énergie du flux d'aspiration en énergie mécanique. La rotation de l'hélice (25) autour de son axe est guidée par l'orifice d'entrée (27) du bloc de refoulement (2). Le nombre de pales peut varier afin d'optimiser le rendement de l'hélice (25). L'hélice (25) se compose d'un minimum de deux pales réunies par la partie centrale appelée moyeu (33). Le couple de l'hélice (25) est en relation avec le flux d'air. L'extrémité des pales a une vitesse linéaire plus importante que les sections de pales plus au centre. L'énergie mécanique produite par l'hélice (25) dépend de trois paramètres: la forme et la longueur des pales, la vitesse du filtrat, puis enfin, la température qui influe sur la densité du filtrat (F). La puissance de l'hélice (25) dépend principalement de l'énergie du filtrat (F) et de ses variations. L'énergie mécanique de l'hélice (25) est donc une énergie intermittente. En pratique, l'hélice (25) produit quatre fois plus d'énergies si les pales sont deux fois plus grandes et huit fois plus d'énergies si le débit d'aspiration double. La densité du filtrat (F) entre également en jeu, l'hélice (25) produit 3% de puissances en plus si, pour une même vitesse le filtrat (F) est plus froid de 10°C. La puissance maximum récupérable par l'hélice (25) est donnée par la loi de Betz, qui est égale au produit de la multiplication de la pression atmosphérique avec la surface balayée et le cube de la vitesse du filtrat (F). De ce fait, la pression atmosphérique, la surface balayée, et la vitesse du filtrat (F) sont proportionnelles entre elles, et varient si l'une d'elles varie. Le filtre (3) comporte au centre du moyeu (33) un conduit fluidique (34) qui collecte par son orifice d'entrée (35) le tissu sain (G) dans le tamis (30) et le conduit à l'orifice (27) du bloc de refoulement (2). Le filtre (3) comporte un épaulement (36), qui a pour but de réduire la surface en contact avec l'assise (26), et ainsi réduire le frottement lors de la rotation du filtre (3). Le filtre (3) vient reposer sur l'assise (26) dudit bloc de refoulement (2). Le filtre (3) peut comprendre des masselottes, qui sont des masses, afin de modifier son équilibre ou son inertie, afin d'augmenter la vitesse.

L'enveloppe (5) qui entoure l'ensemble des blocs techniques (1,2,3) forme les parois (38,38') de la chambre de traitement (A). Ladite enveloppe (5) assure l'essentiel de l'étanchéité de la chambre de traitement (A). Ladite enveloppe (5) présente une zone inférieure (intérieure ou inférieure) et une zone supérieure , ces deux zones sont horizontales.

Ladite enveloppe (5) comporte sur sa zone supérieur moyen d'attache nommé anse (37) servant à saisir ou à porter la chambre de traitement (A).

Ladite enveloppe (5) comporte sur sa zone inférieure deux parois (38,38') collées ou soudées thermiquement avec les blocs (1,2). Cette enveloppe (5) peut être en plastique souple par exemple les mêmes que l'on peut retrouver sur les poches de liquide physiologique. L'enveloppe (5) protège le tissu adipeux (G) des éléments extérieur et plus particulièrement des rayon lumineux, de la poussière et des micro-organismes ou d'agents infectieux.

L'enveloppe (5) peut être d'un matériau configuré pour les basses, les moyennes, les hautes dépressions. Ladite enveloppe (5) peut être également traitée anti-ultraviolets (UVA,UVB) et lumière bleue. Le traitement aux UV est un critère indispensable à la fois pour protéger le tissu adipeux (G) des dangers du soleil et également pour garantir au tissu adipeux (G) une sécurité face aux variations de températures.

Selon une variante, lorsque la chambre de traitement (A) comprend un grand volume de traitement, celle-ci présente un mécanisme qui agite le volume de tissu adipeux (G) en le mouvant, du bas vers le haut, et autour de l'axe (a) de rotation du filtre (3). Ce moyen d'agitation desdits tissus adipeux est autrement nommé train d'engrenages épicycloïdal (6). Un train d'engrenages est qualifié d'épicycloïdal quand il a la particularité d'avoir deux degrés de mobilité. Pendant le fonctionnement, les roues dentées ou les pignons qui composent le train ne tournent pas tous autour d'axes fixes, ce qui leur donne une course épicycloïdal, d'où le nom donné au train (6). Ledit train d'engrenages épicycloïdal (6), situé dans la zone supérieure de la chambre de traitement (A), est logé dans le logement (16) dudit bloc d'admission (1). Constitué d'engrenages, ce train d'engrenages épicycloïdal (6), présente une zone planétaire intérieure, une zone planétaire extérieure, et une zone satellitaire entre les deux précédentes zones, ces trois zones sont coaxiales. Ledit train épicycloïdal (6) comporte deux arbres coaxiaux dits planétaires (8,9), et trois satellites (7,7',7") engrenant avec les deux planétaires (8,9). Ledit planétaire intérieur (8) est parfois appelé soleil, et le planétaire extérieur (9) appelé couronne. Dans cette version, ledit train d'engrenages épicycloïdal (6) de ladite chambre de traitement (A) est composé en plus d'un porte satellites (10), et une goupille (11). De plus, l'anneau (28) du filtre rotatif (3) intègre une couronne à denture intérieure (9), et le conduit (14) du bloc d'admission (1) intègre un méplat (39), un épaulement (40), et un perçage (41). Le mécanisme est logé dans le logement (16) du bloc d'admission (1), entre ledit épaulement (40) du bloc d'admission (1) et ladite goupille (11) logée dans le perçage (41) du conduit fluidique (14).

Ladite couronne (9) du filtre (3) située dans la zone planétaire extérieure dudit train (6) de la chambre de traitement (A), distribue la puissance aux différents agitateurs (7,7',7") de façon équilibrée.

Lesdits agitateurs épicycloïdaux (7,7',7"), situés dans la zone satellitaire dudit train (6) de la chambre de traitement (A), présentent chacun une zone centrale, une zone supérieure et une zone inférieure, ces trois zones sont horizontales. Ces trois agitateurs épicycloïdaux (7,7',7"), sont de dimensions et d'aspect équivalents, mais peuvent présenter des configurations différentes. Les agitateurs épicycloïdaux (7,7',7"), mélangent le tissu adipeux (G) principalement avec deux mouvements, de la périphérie au centre et vice versa, et/ou, du bas vers le haut.

Lesdits agitateurs (7,7',7") sont composés chacun sur leurs zones supérieures d'un arbre (42) en liaison pivot avec le porte satellite (10). Chaque arbre (42) accueille un anneau élastique (12,12',12"). Lesdits anneaux élastiques autrement nommés circlips (12,12',12") sont prévus pour maintenir en position les arbres (42).

Lesdits agitateurs (7,7',7"), sont composés sur leurs zones centrale de trois pignons (43) à denture droite. Les trajectoires des points des pignons (43) par rapport au bloc d'entrée (1) sont des courbes cycloïdales, d'où le nom donné au dispositif. Les dentures des pignons (43) sont en contact avec les dentures des deux planétaires (8,9). Lesdits agitateurs (7,7',7") agitent le tissu (G) à une vitesse inférieure de celle des malaxeurs (32) du filtre (3). Lesdits agitateurs (7,7',7"), sont composés chacun sur leurs zones intérieures d'un fouet (44), pour mélanger ou agiter le retentât (G) composé du tissu prélevé (I), de liquide physiologique (L), et d'air. Lors du fonctionnement du dispositif, lesdits fouets (44) agitent le tissu (G) suivant une trajectoire autour de l'axe (b), et une trajectoire autour des axes (c,c',c"). Lesdits agitateurs épicycloïdaux (7,7',7"), tournent autour de leurs axes (c,c',c") en mouvement par rapport à l'axe (b) du conduit (14). Lesdits agitateurs (7,7',7") tournent sur une roue fixe (8) au bloc d'admission (1), de grande taille, assurant une rotation silencieuse et précise, même dans des conditions de travail difficiles. Ladite roue fixe (8) supporte le porte satellite (10). La rotation de la roue fixe (8) est arrêtée par le méplat (39), sa position verticale est déterminée par la goupille (11) dans le perçage (41).

Lesdits agitateurs (7,7',7"), sont composés sur leurs zones inférieures de trois fouets (44). Lesdits fouets (44) sont en contact avec le tissu adipeux (G), lesdits fouets (44) ont pour fonction d'agiter et séparer les composants du retentât (G). La forme du fouet (44), peut être modifiée pour permettre un meilleur traitement des graisses (G).

Ledit porte satellite (10) est logé entre l'épaulement (40) du bloc d'admission (1) et la roue fixe (8). Ledit porte satellite (10) fonctionne comme une manivelle tournante autour de l'axe (b) et du conduit (14). Ledit porte satellite (10) présente trois manivelles (10') équidistantes de 120 degrés. Chaque manivelle (10') comprend un logement ou viennent se loger les agitateurs (7,7',7"). Lesdites manivelles (10') transforment en mouvement de rotation autour de l'axe (b), une ou plusieurs forces transmises par les agitateurs (7,7',7").

Ladite goupille (11) et son perçage (41) ont pour but de définir les positions horizontales de la roue fixe (8) et du porte satellite (10), et par conséquent, les positions des agitateurs (7,7',7"), tout en garantissant le bon fonctionnement du train épicycloïdal (6). Ladite goupille (11) et son perçage (41) permettent un compromis entre la précision requise pour un fonctionnement satisfaisant dudit train épicycloïdal (6), et les contraintes techniques et économiques de la fabrication.

Ledit conduit (14) du bloc d'admission (1) intègre un méplat (39). Ledit méplat (39), aussi nommé plat, est une surface plane sur le conduit fluidique (14) de forme cylindrique. Ce méplat (39) entrave le mouvement de la roue fixe (8), afin de bloquer volontairement sa rotation.

Un volume de tissu adipeux (G) trop humide ou trop sec peut entrainer l'asphyxie, une ischémie létale des cellules graisseuses adipocytes (G), pouvant amener de mauvais résultats, des désagréments voire des risques sanitaires. De ce fait contrôler le processus durant le traitement peut s'avérer vitale pour les cellules graisseuses adipocytes (G).

Selon une autre variante, ladite chambre de traitement (A) présente alors un ensemble de matériel, de logiciels et de modifications dans la chambre de traitement (A) servant à surveiller ou contrôler le processus de traitements du tissu adipeux (G). Selon ce mode préféré de réalisation, ladite chambre de traitement (A) peut présenter l'intégration de composants électroniques (45,45',46,46'), ainsi qu'un certain nombre de modifications du bloc d'admission (1) relatives à ces composants électroniques. Selon ce mode préféré de réalisation, ladite chambre de traitement (A) peut présenter en plus un moniteur (47) affichant des taux d'humidité et de températures relevés par les composants électroniques (45,45',46,46') de ladite chambre de traitement (A), afin que l'utilisateur puisse contrôler l'état des cellules (G).

Selon ce mode préféré de réalisation, ledit contrôle du processus est assuré dans la chambre de traitement (A), par au moins un composant électroniques actif nommé capteur (45) permettant les mesures d'humidité et de température. Pour faciliter la description, un seul capteur est représenté. Cependant, la chambre de traitement (A) peut être équipée de capteurs de vitesse, de température, d'humidité et de luminosité, conjointement et/ou indépendamment. Ledit capteur (45) recueille les valeurs d'humidités et de températures des adipocytes (G) sans altérer leurs qualités, et les transmet par son circuit électronique (45'). Ledit circuit électronique (45') transmet les données révélées par ledit capteur (45) au moyen de connexion nommé port USB (46) par la connectique (46').

Selon ce mode préféré de réalisation, le bloc d'admission (1) comprend sur sa partie extérieure, un socle (48) pour moyen de contrôle électronique nommé moniteur (47), ainsi qu'un logement (49) pour le port USB (46) 2.0 ou 3.0 mâle. Dans ladite version, le bloc d'admission (1) présente sur sa partie intérieure, un conduit fluidique (14) au dimensions réduites pour laisser place à un conduit électronique (50). Ledit conduit électronique (50) longe le conduit fluidique (14) du logement (49) jusqu'au centre du filtre (3). Ledit conduit électronique (50) permet le passage et le logement du circuit électronique (45') du capteur (45). Le conduit électronique (50) permet également d'éviter tout risque de court-circuit. Lesdits fouets (44), peuvent aussi se décliner en fonction du besoin, ils peuvent à la fois comporter une sonde de température, une sonde d'humidité, de luminosité, ou rester simple.

Selon ce mode préféré de réalisation, ledit moniteur (47) permet la communication du dispositif avec l'utilisateur. Ledit moniteur (47) est composé d'un écran (51) TFT ou LCD qui affiche le résultat d'une mesure. L'affichage peut être analogique, soit avec la particularité d'avoir un système d'indicateurs de températures ou d'humidité qui se fait par l'intermédiaire du déplacement d'une aiguilles sur un cadran, ou numérique, soit avec les indications directement sous la forme de chiffres ou de lettres. Un rétro-éclairage peut être intégré à l'écran (51).

Le moniteur (47), intègre une micro ou nano carte programmable (52) qui possède un micro contrôleur facilement programmable, ainsi que de nombreuses entrées-sorties. Il est utile de préciser que plusieurs cartes existent, elles se différencient par la puissance du micro contrôleur ou par la taille et la consommation de la carte. Le choix du type de carte s'effectue en fonction des besoins. La carte (52) se programme à l'aide d'un logiciel de programmation gratuit.

Le moniteur (47) intègre une batterie (53) lithium-ion, avec une coque rigide ou sous gaine. Ladite batterie (53) lithium-ion permet une autonomie importante avec un volume et un poids limité. Ladite batterie (53) est avec un circuit de protection et ne nécessite aucune protection extérieure contre la surcharge, l'inversion de polarité ou un éventuel défaut du chargeur. De plus, le moniteur (47) peut être directement alimenté par un chargeur dont la tension est comprise entre 7 et 12 volt.

Le moniteur (47) intègre une solution d'habillage avec une coque (54) et un couvercle (54'). Les formes desdits coque (54) et couvercle (54') peuvent être modifiés si besoin. Lesdits coque (54) et couvercle (54') réalisent une protection contre l'humidité ou tout simplement une protection contre les chocs.

Le moniteur (47) peut aussi contenir des sondes de mesures de luminosité, des systèmes d'alerte sonore ou d'éclairage si besoin.

Il est important de préciser que dans cette version du dispositif, les composants électroniques (45,45',46) dans la chambre de traitement (A) dépendent énergétiquement du moniteur (47). Le moniteur (47) peut communiquer avec la chambre de traitement (A) par connexion filaire (USB, jack, fibre optique, etc.). Cependant, d'autres conceptions peuvent permettre à la chambre de traitement (A) d'être indépendante énergétiquement. Selon une variante, la chambre de traitement (A) possède une fonction dynamoélectrique liée à la rotation du filtre (3), ou selon une autre variante la chambre de traitement (A) possède un moteur et une batterie, etc. Ainsi la chambre de traitement (A) peut transmettre les valeurs graphiques à un ordinateur, une imprimante, un Smartphone ou une tablette tactile, par le biais de transmission sans fil (Bluetooth, Wifi, SMS, mail, etc.).

Il convient de rajouter qu'un certain nombre d'améliorations, de fonctionnalités supplémentaires individuelles, ou en combinaison sont applicables à l'invention.

Chacun des éléments peut, sans y être tenu, être utilisé avec tout autre caractéristique ou combinaison. Le dispositif peut être constitué de tout matériau de construction approprié; chaque pièce, composant; ou ensemble, peut être constitué d'une ou plusieurs compositions de plastique. L'appareil peut être configuré avec une taille très pratique pour une utilisation optimale du volume de collecte. L'appareil est conçu pour un nombre d'utilisation défini par l'état du filtre (3). L'appareil peut être dimensionné pour le transport (déplacé à d'autres endroits différents où une variété de traitement du tissu collecté peut être effectuée). L'appareil peut être conditionné dans un environnement stérile et hermétique, dans un emballage pour le transport ou le stockage dans une banque de tissus avant l'utilisation. L'appareil peut être stérilisé avant par la chaleur sèche, par la vapeur d'eau saturée, ou après l'emballage par exposition au plasma. Pour prévenir des risques associés à une utilisation abusive, il peut être découpé avec un ciseau, pour endommager l'appareil d'une manière qui le rend insatisfaisant pour la réutilisation.

L'appareil peut être portable et facilement transportable entre les endroits où les opérations de collecte ou des traitement différents peuvent être menés.

Pour utiliser le dispositif, l'utilisateur a besoin:
- d'un bloc ou salle opératoire (B)
- d'un patient (P) représenté dans les figures par un mannequin sur une table d'opération (Q).
- d'un moyen ou système d'aspiration (M) avec un système de recueil (N).
- d'un pied à perfusion autrement nommé potence (O).
- d'une poche de liquide physiologique (L),
- d'une canule (R) pour permettre l'aspiration sans endommager les tissus.
- Robinet à trois voies (D) en forme de "T"
- de quatre tuyaux chirurgicaux (C,J,E,H,S), avec raccord conique.
- d'une seringue (K) avec raccord luer-lock.

Ledit bloc ou salle opératoire (B) est conçu de manière à optimiser la sécurité du patient et à contribuer aux démarches de prévention des infections. Les salles d'opération bénéficient en règle générale d'équipements de dernière génération. Ces salles profitent de dispositifs médicaux performants et modulables, adaptés pour réaliser des actes chirurgicaux complexes mais aussi pour faciliter l'enseignement universitaire.

Le patient (P) est préparé pour une chirurgie ambulatoire, ou plus simplement, une chirurgie programmée et réalisée dans les conditions techniques nécessitant impérativement la sécurité d'un bloc opératoire, sous une anesthésie de mode variable, suivie d'une surveillance postopératoire permettant, sans risque majoré, la sortie du patient le jour même de son intervention. Ledit acte chirurgical est identique à celui réalisé en chirurgie classique avec hospitalisation complète. Il n'existe pas non plus de spécificité pour l'anesthésie. Ce n'est pas l'acte qui est ambulatoire, mais le patient.

Le fonctionnement de la chambre de traitement (A) repose sur le débit d'air et sur la force centrifuge. De ce fait, la chambre de traitement (A) fonctionne avec tout type de moyen d'aspiration. Cependant, idéalement, et pour une facilité de compréhension, ledit moyen d'aspiration est un plateau technique (M) permettant le raccordement aux prises de fluides médicaux, aux prises électriques, et aux prises Ethernet, des équipements dans les salles d'opération et de soins intensifs. Ledit plateau technique (M) est intégré au bloc opératoire (B). Les plateaux techniques peuvent être configurés en fonction des besoins. Ainsi tout accessoire supplémentaire peut être ajouté au plateau technique (M) tel que des prises électriques, prises de gaz, appels infirmier, interrupteurs de lumière etc. Ledit plateau technique (M) est connecté fluidiquement avec le recueil (N) par un tuyau (S).

Ledit pied à perfusion (O) autrement nommé potence est un élément mobile où l'on accroche une poche ou le flacon à perfusion, afin de permettre au patient de se déplacer. Ledit pied à perfusion (O) est soit à roulettes pour une mobilité accrue ou fixé sur le lit pour un minimum d'encombrement.

Le liquide physiologique (L) est généralement composé d'eau distillée et de chlorure de sodium, et est sans danger pour le corps, il est couramment utilisé à des fins médicales notamment pour le remplissage vasculaire en perfusion intraveineuse, le nettoyage des plaies, ou le maintien provisoire d'organes séparés du corps dans un état propice à l'observation, l'analyse ou la greffe. Le liquide physiologique (L) est improprement nommé sérum physiologique mais ne provient pas directement du sang.

La chambre de traitement (A) fonctionne avec différentes méthodes de prélèvement chez un patient, ou avec des tissus conditionné par une banque de tissus. De ce fait, un petit tube ou tuyau permettant le passage de tissus et liquide à travers un orifice obtenu après intervention chirurgicale sur le patient (P), ou suffit au bon fonctionnement de la chambre de traitement (A). Cependant, et pour une facilité de compréhension, l'utilisation d'une canule (R) de prélèvement est utilisée dans les procédures de transfert de tissus prélevé (I), pendant la phase de prélèvement des tissus adipeux (G) au sein du corps du patient (P). Ladite canule (R) ne présente pas d'angles ou de bords saillants et ne laisse pas de points de pression.

Ledit robinet 3 voies (D) est avec 2 sites d'injections, avec chacun un levier de manœuvre (D',D"). Le premier levier (D') contrôle le débit du prélèvement aspiré, et le second levier (D") contrôle le débit de liquide physiologique. Ledit robinet (D) peut être automatisé, par le biais d'un servomoteur ou assurant la conversion d'un signal de commande en mouvement des leviers de manoeuvre (D',D"). Selon un autre mode de réalisation où le prélèvement de tissus (I) et le liquide physiologique (L) ne se mélangent que dans la chambre de traitement (A), ledit robinet 3 voies (D) est remplacé soit par deux orifices (13) pour le conduit fluidique (14) du bloc d'admission (1), soit par un second conduit fluidique identique au conduit fluidique (14).

Lesdits tuyaux chirurgicaux (C,J,E,H,S) conservent parfaitement leurs formes pendant l'aspiration. Chaque extrémité est dotée de raccords femelles universels pour une fixation pratique et sécurisée. Deux tuyaux (C,J) servent respectivement à connecter fluidiquement la chambre de traitement (A) avec la sortie du robinet (D) et le recueil (N) du plateau technique (M), ainsi que deux tuyaux (E,H) qui servent respectivement à connecter fluidiquement les entrées du robinet (D) avec le liquide physiologique (L) et la canule (R) d'aspiration.

Pour la préparation, il convient de respecter les étapes suivantes: installer la chambre de traitement (A) par son anse (37) sur la potence (O). Installer le sérum physiologique (L) sur la potence (O). Raccorder un premier tuyau (C) à l'orifice d'entrée (13). Raccorder le tuyau (J) du recueil (N) à l'orifice de sortie du filtrat (17) de la chambre de traitement (A). Raccorder la seringue luer-lock (K) à l'orifice de sortie luer-lock (19), de la chambre de traitement (A). Vérifier que les leviers (D') et (D") du robinet (D) sont fermés. Raccorder le tuyau (C) à l'orifice de sortie de la vanne (D). Raccorder le sérum physiologique (L) sur un premier orifice d'entrée du robinet (D), à l'aide du tuyau (E). Raccorder le tuyau (H) de la canule d'aspiration sur un second orifice d'entrée du robinet (D). Installer sur le socle (48) le moniteur (47). Allumer le moniteur (47). Actionner l'aspiration.

La chambre de traitement (A) peut comprendre une fonction mécanique de marche/arrêt de la centrifugation du filtre (3) située à l'extérieure.

L'ouverture totale du levier (D') du robinet (D) permet à l'utilisateur de balayer avec la canule (R) la zone où le tissu (I) doit être prélevée, et permet d'actionner mécaniquement la chambre de traitement (A). Sous l'effet du flux d'aspiration, le tissu prélevé (I) délogé est aspiré loin à travers le tuyau (H) jusqu'au robinet (D). Sous l'effet du flux d'aspiration l'hélice (25) s'actionne et transforme l'énergie du flux d'aspiration en énergie mécanique, en mouvement de rotation. Débute alors la phase de centrifugation.

L'ouverture partielle du levier (D") du robinet (D) permet au liquide physiologique (L) de se diriger le long du tuyau (E), sous l'effet de la gravité et de la dépression, jusqu'au robinet (D). L'ouverture partielle du levier (D") permet aussi de contrôler le débit du liquide physiologique (L). Le tissu prélevé (I) et le liquide physiologique (L) se mélangent dans le robinet (D) pour ressortir dans le tuyau (C). Le tuyau (C) dirige le mélange "liquide physiologique (L)/tissu prélevé (I)" vers l'orifice (13) de la chambre de traitement (A). Le mélange "liquide physiologique (L)/tissu prélevé (I)" traverse le conduit (14), puis se dépose dans le tamis (30) du filtre (3). Le filtre (3) à l'aide des malaxeurs (32) agite le mélange "liquide physiologique (L)/tissu prélevé (I)" et ne conserve que les graisses ou tissus adipeux (G). De plus le sérum physiologique (L), à la fois hydrophile et hydrophobe, se fixe sur les matières organiques que l'eau seule ne peut retirer et les évacuent a travers le tamis (30) sous l'effet de l'inertie. Le tamis (30) confine un retentât (G), et libère les fluides de nettoyage (L) et matières de tailles inférieures. Lorsque la chambre de traitement (A) comprend un train épicycloïdal (6), les agitateurs (7,7',7") agitent le tissu adipeux (G) à l'intérieur du filtre (3). Ce qui améliore son traitement et son rinçage. Le mélange évacué a travers les mailles du tamis (30) est nommé filtrat (F). Le filtrat (F) est emporté de l'extérieur du tamis (30), vers le conduit fluidique (18) dudit bloc de refoulement (2), puis évacué par le l'orifice de sortie (17) dans le tuyau (J) jusqu'au recueil (N) du plateau technique (M). Sous l'effet de la gravité le filtrat (F) tombe dans le conduit (18). Le filtrat (F) est ensuite évacué en continu. Dans sa course, le filtrat (F) transmet intégralement les efforts a l'hélice (25), ce qui réduit le couple, augmente la vitesse nominale de rotation du filtre (3). Le filtrat (F) est dirigé le long du conduit (18) vers l'orifice (17), pour rejoindre le tuyau (J). Le filtrat (F) est dirigé le long du conduit (18) sous l'effet de la gravité et de la dépression.

Un petit volume de tissus adipeux (G') est dirigé le long du conduit (20), ce volume n'est pas ou peu traité, il devra donc être retiré puis jeté.

Une fois le volume requis de tissu (G) atteint, laisser les leviers (D') et (D") du robinet (D) ouvert, jusqu'a ce que le filtrat (F) soit composé en majorité de sérum physiologique (L). Le bloc d'admission (1) peut comprendre une ouverture avec un filtre anti bactérien pour augmenter le flux dans la chambre de traitement (A) et la vitesse du filtre (3). Lorsque le filtrat (F) est composé en majorité par le sérum physiologique (L), fermer le levier (D"). Dans ladite version ou le dispositif comprend un moniteur (47), contrôler le taux d'humidité et la température relevée par le capteur (45) sur le moniteur (47).Tout au long de l'opération de traitement du tissu adipeux (G), l'utilisateur peut contrôler l'état du tissu adipeux (G) sur le moniteur (47).

À satisfaction de l'état, fermer le levier (D') du robinet (D). Arrêter le système d'aspiration. Aspirer le volume de tissus (G') non traité logé dans le conduit (20), à l'aide de la seringue (k). Vider le contenu de la seringue (K) dans un récipient adapté. Insérer la seringue (k) dans le port luer-lock et extraire le retentât (G) contenu dans la chambre de traitement (A), afin de le greffer. La forme conique du filtre (3) permet de diriger le tissu (G) en son sein, et de bénéficier de 80% à 95% du volume traité disponible. L'utilisation d'une chambre de traitement (A) est de un donneur pour plusieurs receveurs. De ce fait, l'utilisateur peut faire plusieurs prélèvements de tissus sur un donneur, et les regreffer sur des receveurs différents.

Après l'usage, l'appareil peut être mis au déchet ou au tri sélectif de façon appropriée.

De nombreuses variantes éventuellement susceptibles de se combiner peuvent ici être apportées sans jamais sortir du cadre de l'invention tel qu'il est défini ci-avant.

### Applications industrielles

L'appareil peut être utilisé pour des applications variées de traitement impliquant des tissus adipeux.

Le dispositif peut être utilisé pour des applications variées comme le lipofilling, la construction-reconstruction mammaire, le comblement de cicatrices etc.

Le dispositif peut être utilisé pour le traitement avant analyse du tissu adipeux et peut permettre l'exécution de plusieurs opérations différentes lorsqu'il est en fonction.

Le dispositif peut être utilisé pour la préparation de concentrés, ou en vue de séparer les parties du tissu prélevé.

## Revendications

1. Dispositif de traitement de tissus adipeux (G) qui est connectable à un moyen d'aspiration (M) **caractérisé en ce qu'**il est constitué d'une chambre de traitement (A) mécanique et étanche, comprenant un moyen de connexion (17) audit moyen d'aspiration (M), un moyen d'entrée (13) desdits tissus adipeux (G) avant traitement, un moyen de filtration (3) et au moins un moyen de sortie (20) desdits tissus adipeux (G) après traitement, ledit moyen de filtration (3) comprenant au moins une paroi de forme conique (31,31') comportant une base resserrée (55) fixée sur une hélice (25) et un moyen de tamisage (30) relié au moyen de sortie (20), ladite hélice (25) étant soumise à la dépression d'air créée par le moyen d'aspiration (M) et constituant un moyen d'actionnement mécanique rotatif dudit moyen de filtration (3).

2. Dispositif de traitement de tissus adipeux (G) selon la revendication 1 **caractérisé en ce que** le moyen d'entrée (13) desdits tissus adipeux (G) constitue un moyen d'entrée d'un fluide de nettoyage (L).

3. Dispositif de traitement de tissus adipeux (G) selon la revendication 2 **caractérisé en ce que** la chambre de traitement (A) comporte un moyen de sortie du fluide de nettoyage (L) et matières de tailles inférieures au maillage du moyen de tamisage (30).

4. Dispositif de traitement de tissus adipeux (G) selon la revendication 1 **caractérise en ce que** le moyen de tamisage (30) est constitué d'un maillage, chacune des mailles constituant ledit maillage ayant une dimension comprise entre 500 microns et 1 mm.

5. Dispositif de traitement de tissus adipeux (G) selon la revendication 1 **caractérisé en ce que** le moyen de filtration (3) comporte au moins un moyen de malaxage (32).

6. Dispositif de traitement de tissus adipeux (G) selon la revendication 1 **caractérisé en ce que** le dispositif de traitement de tissus adipeux (G) est connecté à un moyen d'aspiration (M) par raccordement d'un tuyau (J) du moyen d'aspiration (M) au moyen de connexion (17) de ladite chambre de traitement (A).

7. Dispositif de traitement de tissus adipeux (G) selon l'une des revendications précédentes **caractérisé en ce que** la chambre de traitement (A) peut comprendre un train épicycloïdal (6) constituant un moyen d'agitation desdits tissus adipeux (G).

8. Dispositif de traitement de tissus adipeux (G) selon l'une des revendications précédentes **caractérisé en ce que** la chambre de traitement (A) peut comprendre au moins un composant électronique actif (45) reliée par un moyen de connexion a un moyen de contrôle électronique (47) du processus de traitement des tissus adipeux (G).

9. Dispositif de traitement de tissus adipeux (G) selon l'une des revendications précédentes **caractérisé en ce que** la chambre de traitement (A) est délimité par un matériau souple (5) protégeant le tissu adipeux (G) des éléments extérieur.

10. Dispositif de traitement de tissus adipeux (G) selon l'une des revendications précédentes **caractérisé en ce que** la chambre de traitement (A) comporte un moyen d'attache (37).

## Patentansprüche

1. Vorrichtung zur Behandlung von Fettgeweben (G), die mit einem Ansaugmittel (M) verbunden werden kann, **dadurch gekennzeichnet, dass** sie aus einer mechanischen und dichten Behandlungskammer (A) besteht, umfassend ein Verbindungsmittel (17) am Ansaugmittel (M), ein Eingangsmittel (13) für die Fettgewebe (G) vor der Behandlung, ein Filtriermittel (3) und mindestens ein Ausgangsmittel (20) für die Fettgewebe (G) nach der Behandlung, wobei das Filtriermittel (3) mindestens eine konisch geformte Wand (31, 31') umfasst, die eine auf einer Schraube (25) befestigte eingezwängte Basis (55) umfasst und ein Siebemittel (30), das mit dem Ausgangsmittel (20) verbunden ist, wobei die Schraube (25) dem durch das Ansaugmittel (M) gebildeten Luftunterdruck ausgesetzt ist und ein drehbares mechanisches Auslösemittel des Filtriermittels (3) bildet.

2. Vorrichtung zur Behandlung von Fettgeweben (G) gemäß Anspruch 1, **dadurch gekennzeichnet, dass** das Eingangsmittel (13) für die Fettgewebe (G) ein Eingangsmittel für eine Säuberungsflüssigkeit (L) bildet.

3. Vorrichtung zur Behandlung von Fettgeweben (G) gemäß Anspruch 2, **dadurch gekennzeichnet, dass** die Behandlungskammer (A) ein Ausgangsmittel für die Säuberungsflüssigkeit (L) und Stoffe mit kleinerer Vernetzung als das Siebemittel (30) umfasst.

4. Vorrichtung zur Behandlung von Fettgeweben (G) gemäß Anspruch 1, **dadurch gekennzeichnet, dass** das Siebemittel (30) aus einer Vernetzung besteht, wobei jede der Maschen, welche die Vernetzung bilden, eine Dimension im Bereich zwischen 500 µm und 1 mm hat.

5. Vorrichtung zur Behandlung von Fettgeweben (G) gemäß Anspruch 1, **dadurch gekennzeichnet, dass** das Filtriermittel (3) mindestens ein Rührmittel (32) umfasst.

6. Vorrichtung zur Behandlung von Fettgeweben (G) gemäß Anspruch 1, **dadurch gekennzeichnet, dass** die Vorrichtung zur Behandlung von Fettgeweben (G) mit einem Ansaugmittel (M) verbunden ist, durch Anschluss an ein Rohr (J) des Ansaugmittels (M) an das Verbindungsmittel (17) der Behandlungskammer (A).

7. Vorrichtung zur Behandlung von Fettgeweben (G) gemäß irgendeinem der vorhergehenden Ansprüche, **dadurch gekennzeichnet, dass** die Behandlungskammer (A) ein Planetengetriebe (6) umfassen kann, welches ein Vermischungsmittel der Fettgewebe (G) bildet.

8. Vorrichtung zur Behandlung von Fettgeweben (G) gemäß irgendeinem der vorhergehenden Ansprüche, **dadurch gekennzeichnet, dass** die Behandlungskammer (A) mindestens einen aktiven elektronischen Bestandteil (45) umfassen kann, verbunden über ein Verbindungsmittel ein elektronisches Steuerungsmittel (47) des Behandlungsverfahrens der Fettgewebe (G).

9. Vorrichtung zur Behandlung von Fettgeweben (G) gemäß irgendeinem der vorhergehenden Ansprüche, **dadurch gekennzeichnet, dass** die Behandlungskammer (A) durch ein weiches Material (5) abgegrenzt wird, welches das Fettgewebe (G) vor den äußeren Elementen schützt.

10. Vorrichtung zur Behandlung von Fettgeweben (G) gemäß irgendeinem der vorhergehenden Ansprüche, **dadurch gekennzeichnet, dass** die Behandlungskammer (A) ein Befestigungsmittel aufweist.

## Claims

1. Device for treating adipose tissues (G) connectable to a suctioning means (M), **characterised in that** it consists of a mechanical and hermetically sealed treatment chamber (A), comprising a connection means (17) to said suctioning means (M), an input means (13) for said adipose tissues (G) before processing, a filtration means (3) and at least one output means (20) for said adipose tissues (G) after processing, the said filtration means (3) comprising at least one conically shaped wall (31,31') comprising a narrowed base (55) fixed on an impeller (25) and a screening means (30) connected to the output means (20), wherein said impeller (25) is subjected to the air vacuum created by the suctioning means (M) and forming a rotating mechanical activation means of said filtration means (3).

2. Device for treating adipose tissues (G) according to claim 1, **characterised in that** the input means (13) for said adipose tissues (G) forms an input means for a cleaning fluid (L).

3. Device for treating adipose tissues (G) according to claim 2, **characterised in that** the treatment chamber (A) comprises an output means for cleaning fluid (L) and material of sizes smaller than the mesh of the screening means (30).

4. Device for treating adipose tissues (G) according to claim 1, **characterised in that** the screening means (30) consists of a screen, wherein each of the meshes forming said screen has a dimension between 500 microns and 1 mm.

5. Device for treating adipose tissues (G) according to claim 1, **characterised in that** the filtration means (3) comprises at least one mixing means (32).

6. Device for treating adipose tissues (G) according to claim 1, **characterised in that** the device for treating adipose tissues (G) is connected to a suctioning means (M) by attachment to a pipe (J) of the suctioning means (M) by a connection (17) of the said treatment chamber (A).

7. Device for treating adipose tissues (G) according to any one of the previous claims, **characterised in that** the treatment chamber (A) may include a planetary set (6) comprising an agitation means for said adipose tissues (G).

8. Device for treating adipose tissues (G) according to any one of the previous claims, **characterised in that** the treatment chamber (A) may comprise at least one active electronic component (45) connected by a connection means to an electronic control means (47) of the treatment method of the adipose tissues (G).

9. Device for treating adipose tissues (G) according to any one of the previous claims, **characterised in that** the treatment chamber (A) is delimited by a flexible material (5) protecting the adipose tissue (G) from the external elements.

10. Device for treating adipose tissues (G) according to any one of the previous claims, **characterised in that** the treatment chamber (A) includes an attachment means (37).
